# EUROPEAN PATENT APPLICATION

(11) **EP 2 033 630 A1**
(43) Date of publication of application: **11.03.2009**
(21) Application number: 08012303.7
(22) Date of filing: 08.07.2008
(51) Int. Cl.: A61K 9/20, A61K 9/16, A61K 31/455

(54) **A novel controlled release-niacin formulation**

(30) Priority: 07.09.2007 KR 20070091181
(71) Applicant: Seoul Pharm. Co. Ltd., Seoul 137-070 (KR)
(72) Inventor: Choi, Youn Woong, Ansan-si, Gyeonggi-do 463-865 (KR); Ryoo, Byung Hwan, Seongnam-si, Gyeonggi-do 463-845 (KR); Jeong, Yong Mi, Seongbuk-gu, Seoul 136-102 (KR); Jang, Jae Sang, Yeonsu-gu, Incheon 460-050 (KR)
(74) Representative: Kador & Partner

(57) **Abstract**

The present invention relates to a controlled-release niacin formulation. In particular, the present invention relates to a controlled-release niacin formulation, comprising niacin; hydroxypropyl methylcellulose; and a carboxyvinyl polymer, in which the carboxyvinyl polymer and hydroxypropyl methylcellulose are contained in a predetermined weight ratio, and to a preparation method thereof.

The controlled-release niacin formulation according to the present invention maintains its matrix shape until completion of release, and maintains its release pattern without fluctuation for a desired time period, unlike a commercial formulation. In particular, since niacin formulations are used for long-term treatment of hyperlipidemia, the controlled-release niacin formulation of the present invention, capable of maintaining effective blood concentration and high stability for a long period of time, is very useful.

## Description

### [Technical Field]

The present invention relates to a controlled release oral formulation of niacin. In particular, the present invention relates to a controlled-release niacin formulation, comprising niacin useful for the treatment of hyperlipidemia; hydroxypropyl methylcellulose; and a carboxyvinyl polymer, in which upon oral administration, the hydroxypropyl methylcellulose absorbs moisture to form a water soluble matrix system, thereby controlling the drug release, the carboxyvinyl polymer controls the drug release depending on pH, and the hydroxypropyl methylcellulose and carboxyvinyl polymer are mixed in a predetermined ratio. Thus, the controlled-release niacin formulation of the present invention maintains its matrix shape until completion of release, and maintains its release pattern without fluctuation for a desired time period, unlike other commercial formulations. In addition, the drug release can be delicately controlled in the gastrointestinal tract to improve bioavailability. Accordingly, the controlled-release niacin formulation of the present inventionmaintains its bioavailability within the desired ranges, which is useful for long-term treatment of hyperlipidemia, while reducing side effects commonly associated with niacin formulations.

### [Background Art]

Hyperlipidemia or an elevation in serum lipids is associated with an increase incidence of cardiovascular disease and atherosclerosis.
Several types of hypolipidemic agents have been developed to treat hyperlipidemia, hypercholesteremia or normolipidemics diagnosed with cardiovascular disease. In general, these agents act (1) by reducing the production of the serum lipoproteins or lipids; or (2) by enhancing their removal from the serum or plasma.

In the past, there have been numerous methods proposed for reducing elevated cholesterol levels and for increasing HDL-cholesterol levels. Typically, these methods include diet and/or daily administration of lipid-altering or hypolipidemic agents. Another method proposed concerns periodic plasma dilapidation by a continuous flow filtration system, as described in US Patent No. 4,895,558.

Drugs that lower the concentration of serum lipoproteins or lipids include inhibitors of HMG-CoA reductase, which is the rate controlling enzyme in the biosynthetic pathway of cholesterol. However, in long-term treatment of patients with hyperlipidemia, HMG-CoA reductase inhibitors are known to induce rhabdomyolysis, sex hormone formation disorder, hepatotoxicity, and to cause harm to the fetus when administered to pregnant women.

Other drugs which lower serum cholesterol include, for example, nicotinic acid, bile acid sequestrants, e.g., cholestyramine, colestipol DEAESephadex (Secholex™ and Polidexide™, probucol and related compounds as disclosed in U.S. Patent No. 3.674.836, lipostabil (Rhone-Poulanc), Eisai E5050 (an N-substituted ethanolamine derivative), imanixil (HOE-402), tetrahydrolipstatin (THL), isitigmastanyl phosphorylcholine (SPC, Roche), aminocyclodextrin (Tanabe Seiyoku), Ajinomoto AJ-814 (azulene derivative), melinamide (Sumitomo), Sandoz 58-035, American Cyanimid CL-277, 082 and CL-283, 546 (disubstituted urea derivatives), ronitol (which has an alcohol which corresponds to nicotinic acid), neomycin, p-aminosalicylic acid, aspirin, quarternary amine poly diallyldimethylammonium chloride and ionenes such as disclosed in U.S. Patent No. 4,027,009, poly diallylmethylamine derivatives such as disclosed in U.S. Patent No. 4,759,923, omega-3-fatty acids found in various fish oil supplements, fibric acid derivatives, e.g., gemfibrozil, clofibrate, bezatibrate, fenofibrate, ciprofibrate and clinotibrate, and other known serum cholesterol lowering agents such as those described in U.S. Patent No. 5,200,424, European Patent Application No. 0065835A1, European Patent No, 164-698-A, G.B. Patent No. 1,586,152, and G.B. Patent Application No. 2162-179-A.

Nicotinic acid, also known as niacin, has been used for many years in the treatment of hyperlipidemia or hypercholesteremia. This compound has long been known to exhibit the beneficial effects of reducing total cholesterol, VLDL-cholesterol and VLDL-cholesterol remnants, LDL-cholesterol, triglycerides and apolipoprotein, known as "Lp(a)," in the human body, while increasing desirable HDL-cholesterol.

Nicotinic acid has been normally administered three times per day after meals. This dosing regimen is known to provide a very beneficial effect on blood lipids as discussed in Knopp et al. ["Contrasting Effects of Unmodified and Time-release Forms of Niacin on Lipoprotein in Hyperlipidemic Subjects: Clues to Mechanism of Action of Niacin":Metabolism (34) 7:642-647(1985)]. While such a regimen does produce beneficial effects, cutaneous flushing and the like still often occurs in the hyperlipidemics to whom the nicotinic acid is administered.

In order to avoid or reduce the cutaneous flushing resulting from nicotinic acid therapy, a number of agents have been suggested for administration with an effective antihyperlipidemic amount of nicotinic acid, such as guar gum as reported (US Patent No. 4,965,252), mineral salts (US Patent No. 5,023,245), inorganic magnesium salts (US Patent No. 4,911,917), and non-steroidal anti-inflammatories, such as aspirin (PCT Application No. 96/32942). These agents have been reported to avoid or reduce the cutaneous flushing side effect commonly associated with nicotinic acid dividend dose treatment.

Another method of avoiding or reducing the side effects associated with immediate release niacin is the use of sustained release formulations. Sustained release formulations are designed to slowly release the active ingredient from the tablet or capsule, which allows a reduction in dosing frequency as compared to the typical dosing frequency associated with conventional or immediate dosage forms. The sustained drug release reduces and prolongs blood levels of the drug, and thus minimizes or lessens the cutaneous flushing side effects that are associated with conventional or immediate release niacin products. Sustained release formulations of niacin have been developed, such as Nicobid™ capsules (Rhone-Poulenc Rorer), Endur-acin™ (Innovite Corporation), and a sustained release niacin formulation containing two different types of hydroxypropyl methylcelluloses (hereinafter, abbreviated to 'HPMC') and a hydrophobic component (US Patent Nos. 5,126,145 and 5,268,181). Another sustained release niacin formulation is Niaspan^{®}(MERCK) containing a swelling agent, a binder and a lubricant in addition to the drug.

However, in the conventional sustained release formulations, a swelling agent such as HPMC merely absorbs moisture to form a water soluble matrix, thereby delaying drug release. Thus, since the sustained release formulations do not provide an additional control system for pH environment in the gastrointestinal tract, the drug release is not separately controlled in the stomach and intestine.

Accordingly, in order to solve the problems, the present inventors have developed a controlled-release niacin formulation, comprising an effective amount of niacin; a pH-dependent polymer base; a pH-independent polymer base; an additive; a disintegrant; and a lubricant (Korean Patent Publication No. 593252, Jun, 19, 2006), in which the formulation has a separate release control system for the gastrointestinal tract to delicately control the drug release, in particular, effectively in the intestine, while having the advantage of the water soluble matrix in the conventional sustained release formulations.
Meanwhile, to achieve the delicate and constant control of drug release, the controlled-release niacin formulation should control the release rate within the desired ranges, and also maintain its release pattern without fluctuation until completion of release. In particular, since niacin formulations are used for long-term treatment of hyperlipidemia, there is a need to develop a controlled-release niacin formulation, capable of maintaining effective blood concentration and high stability for a long period of time.

Theoretically, in the sustained release formulations, polymers evenly dispersed in the formulation form a network as a matrix for the control of drug release, so that initial release and release pattern should be constantly controlled for the safety of drug formulation. However, in the known controlled-release niacin formulations, the water soluble matrix releases the effective ingredient, niacin due to irregular erosion. Thus, there are disadvantages in that the formulation does not maintain its own shape to cause irregular release pattern, the risk of unexpected drug release (e.g., dose-dumping) is increased to cause undesirable side effects of niacin, the release of effective ingredient can be completed before the desired time, and constant bioavailability cannot be expected at each time point and in each formulation and subject. Despite the above problems, disclosed are only the sustained-release niacin formulations, in which polymers such as HPMC and/or carboxyvinyl polymer are used singly or as a mixture thereof to form a water soluble matrix, thereby sustaining the drug release, and there is no mention of methods for maintaining the matrix shape for a desired time period.

### [Disclosure]

### [Technical Problem]

The present inventors have conducted extensive studies on the controlled-release niacin formulations. They found that a pH-independent polymer, HPMC and a pH-dependent polymer, carboxyvinyl polymer are mixed in a predetermined ratio to prepare an excellent controlled-release niacin formulation, which maintains its matrix pattern until completion of release, thereby completing the present invention.

### [Technical Solution]

In order to solve the problems, the present invention provides a novel controlled-release niacin formulation having a release control system which separately acts in the stomach and intestine, and maintaining its matrix pattern until completion of release, while having the advantage of a water soluble matrix, and a preparation method thereof.

### [Description of Drawings]

Figs. 1 to 4 are graphs showing the dissolution rate of 500 mg of niacin-containing formulation according to the type of polymer base under various pH conditions, in which Fig. 1 is a graph showing the dissolution rate in artificial gastric juice (pH 1.2), Fig. 2 in an acetic acid buffer solution (pH 4.0), Fig. 3 in artificial intestinal juice (pH 6.8), and Fig. 4 in water;
Fig. 5 is a photograph showing the niacin tablet (500 mg) according to the present invention and a commercial tablet, Niaspanor™ (500 mg, MERCK) before the swelling test;
Figs. 6 and 7 are photographs showing a commercial tablet, Niaspanor™ (Fig. 6) and the niacin tablet according to the present invention (Fig. 7) after the swelling test;
Fig. 8 is a graph showing the dissolution profile, after dissolution test in water for niacin tablets prepared by varying the viscosity of HPMC;
Fig. 9 is a graph showing the dissolution profile, after dissolution test in water for niacin tablets prepared by varying the content of HPMC;
Fig. 10 is a graph showing the dissolution profile, after dissolution test in artificial intestinal juice for niacin tablets prepared by varying the content of carboxyvinyl polymer;
Fig. 11 is a graph showing the dissolution profile, after dissolution test for various drugs having the same composition ratio of carboxyvinyl polymer to HPMC; and
Fig. 12 is a graph showing the time maintaining the matrix shape of niacin tablets, prepared by varying the composition ratio of carboxyvinyl polymer to HPMC.

### [Best Mode]

In one embodiment to achieve the obj ect, the present invention relates to a controlled-release niacin formulation, comprising niacin;hydroxypropylmethylcellulose;and a carboxyvinylpolymer, in which the carboxyvinyl polymer and hydroxypropyl methylcellulose are contained in a predetermined weight ratio.

As used herein, the term "niacin" encompasses nicotinic acid and derivatives thereof, for example, nicotinic acid, nicotinamide, nicotyl alcohol tartrate, and d-glucitol hexanicotinate, and includes all compounds convertible into nicotinic acid by in vivometabolism. The amount of niacin contained in the controlled release formulation of the present invention may be suitably selected in terms of economic considerations and its stability, and is generally 300 to 1000 mg, preferably 500 to 1000 mg (per one daily dose).

"Hydroxypropyl methylcellulose", which is used as a pH-independent polymer base in the controlled release formulation of the present invention, is also called HPMC, and commercially available from Dow Chemical company under the trade name of Methocel. HPMC is available in various grades. In the composition of the present invention, any commercially available HPMC may be employed, and all mentioned in the known arts, for example, EP 375156, US 4,369,172, US 4,357,469, US 4,226,846 and US 4,389,393, are included. The preparation methods of HPMC are well known in the related art. HPMC used in the present invention has a viscosity of preferably 80,000 to 120, 000 cps, and more preferably 100,000 cps.

In addition, the carboxyvinyl polymer, which is used as a pH-dependent polymer base in the controlled release formulation of the present invention, is known as "carbomer" or carboxypolymethylene, and is purchased from commercially available sources, for example, Noveon, Inc. (Cleveland, Ohio) (under the trade name of carbopol^{®}). Carbopol polymers are crosslinked, acrylic acid-based polymers, and are cross-linked with allyl sucrose or allyl pentaerythritol. Carbopol copolymers are polymers of acrylic acid, modified by C₁₀₋₈₀ alkyl acrylates, and crosslinked with allyl pentaerythritol.
The carboxyvinyl polymer includes carbomer 910, 934, 934P, 940, 971P, 974P, 1342 or the like, but the prevent invention is not limited thereto, and may include all types of carboxyvinyl polymers. Preferred carboxyvinyl polymers are selected from the group consisting of carbomer 934P, 971P and 974P. In a specific embodiment, the present inventors used Carbopol 934P NF, Carbopol 971NF, Carbopol 974P NF and Carbopol 71G NF. In the present invention, the carboxyvinyl polymer is used in an amount of 0.3% to 10% by weight, based on the total weight.

The HPMC and carboxyvinyl polymer used in the present invention are contained in the controlled-release niacin formulation of the present invention in a predetermined ratio, so that without erosion, the matrix maintains its own shape capable of exhibiting a constant release rate and pattern during a desired time period when its efficacy maintains, thereby achieving the controlled release of niacin. The controlled-release niacin formulation of the present invention comprises the carboxyvinyl polymer and HPMC in a weight ratio of preferably 1:1 to 1:100, more preferably 1:1.5 to 1:50, and most preferably 1:1.5 to 1:20.

In one preferred embodiment, the controlled-release niacin formulation of the present invention further comprises one or more ingredients selected from the group consisting of an additive, a disintegrant, and a lubricant.

In the present invention, the disintegrant is used to absorb moisture and facilitate the release of niacin, and examples of the disintegrant used in the formulation of the present invention may include one or a mixture thereof selected from the group consisting of croscamellose sodium, sodium starch glycolate, pregelatinized starch [Starch 1500^{®} or Prejel^{®}], microcrystalline cellulose, crospovidone (cross-linked povidone), and other commercially available polyvinylpyrrolidone (PVP, Povidone^{®}), low substituted hydroxypropylcellulose, alginic acid, carboxymethylcellulose, calcium salts and sodium salts, colloidal silicon dioxide (fumed silica, colloidal sillica), guar gum, magnesium aluminium silicate, methyl cellulose, powdered cellulose, starch and sodium alginate.

As the disintegrant, it is preferable to use croscamellose sodium, sodium starch glycolate, pregelatinized starch, microcrystalline cellulose or crospovidone, and commercially available polyvinylpyrrolidone.

As the disintegrant, it is more preferable to use crospovidone, sodium starch glycolate, or microcrystalline cellulose, and it is most preferable to use a mixture of two or more thereof. In this connection, the disintegrant is preferably used in an amount of 5 to 200 parts by weight, and more preferably in an amount of 10 to 100 parts by weight.

In addition, the lubricant used in the controlled-release niacin formulation of the present invention is used for enhancing the moldability of oral formulation. Examples thereof may include magnesium stearate, silica oxide (SiO2) or colloidal silicon dioxide (colloidal silica, Cab-O-SIL^{®}) or talc, but are not limited thereto. The lubricant is preferably used in an amount of 0.1 to 20 parts by weight.

The controlled-release niacin formulation of the present invention may include a pharmaceutically acceptable additive. Examples of the additive may include lactose, sugar, mannitol, lactose and sorbitol. If necessary, the controlled-release niacin formulation of the present invention may further include a preservative, a stabilizing agent or the like.
In one preferred embodiment, the controlled-release niacin formulation of the present invention may include a binder, if necessary. As the binder, any known binder may be used without limitations, and preferably selected from polymers having a repeating unit of 1-ethenyl-2-pyrrolidinone. The polymer generally has a molecular weight of 10,000 to 700,000, known as "povidone".

The binder in Examples of the present invention is preferably used in an amount of 1 to 20 parts by weight, and more preferably in an amount of 10 to 20 parts by weight.

In another embodiment, the present invention provides a method for preparing the controlled-release niacin formulation. In particular, the oral formulation may be prepared using the composition of the present invention according to a known method, for example, wet or dry granulation method, but is not limited thereto. The wet and dry methods are classified by the granulation method of raw material. The dry method is a method for pulverizing and sieving a slug or sheet material prepared by using a device such as a slug machine and a roller compactor, and then mixing with lubricants to perform compression. Further, the wet method is a method for compressing wet granules prepared by adding active ingredients, and is a commonly used method. The moist granules are prepared by extrusion granulation, crushing granulation, dried and sieved, and then tableted by adding a lubricant, if necessary, a disintegrant. The wet and dry methods are well known to those skilled in the art.

In one preferred embodiment, the present invention relates to a method for preparing a controlled-release niacin formulation, comprising the steps of
(a) mixing niacin; hydroxypropyl methylcellulose; a carboxyvinyl polymer; an additive; and a disintegrant;
(b) preparing wet granules by adding a liquid solvent; and
(c) mixing the wet granules with a lubricant to perform tableting,
wherein the carboxyvinyl polymer and hydroxypropyl methylcellulose are mixed in a predetermined weight ratio.
The weight ratio of carboxyvinyl polymer to HPMC is preferably 1:1 to 1:100, more preferably 1:1.5 to 1: 50, and most preferably 1:1.5 to 1:20.
Upon preparing the controlled-release niacin formulation of the present invention, the solvent added to the powder blend includes all solvents which does not affect the activity of active ingredient, niacin and is generally used in the preparation of granule. The solvents are well known in the art. Examples thereof may include, but are not limited to, one or a mixture thereof selected from the group consisting of water, ethanol, isopropyl alcohol, glycerin, propylene glycol and polyethylene glycol. As the liquid solvent, it is preferable to use ethanol or a mixed solvent of water and ethanol. At this time, in the case where the solvent is water alone or a mixed solvent of water and ethanol, it is preferably used in an amount of 5 to 40% by weight, more preferably 10 to 23% by weight, based on the total weight of the drug.Further,in one preferred embodiment,the preparation method of the present invention may further include a step of mixing with a binder in step (a).
In one specific embodiment, the present inventors uniformly mixed niacin, HPMC, a carbomer, an additive and a disintegrant, and added a small amount of liquid solvent thereto. Then, they mixed them to prepare moistured and dried wet granules, and then dried and milled the wet granules. Subsequently, they mixed the resultant with a lubricant and/or a binder, and prepared the formulation by direct tableting using a general tablet press.

As described in the following Examples, the present inventors prepared niacin formulations by varying the ratio of carboxyvinyl polymer to HPMC, and then observed whether the formulations maintain their matrix shape or not. As a result, in the case where the niacin formulations contain the carboxyvinyl polymer and HPMC in a weight ratio of 1:1 to 1:100, the formulations were found to maintain their matrix shape over 24 hrs, unlike other commercial formulations. Consequently, the controlled-release niacin formulation according to the present invention was found to more effectively control the release of drug, as compared to other commercial formulations, thereby significantly reducing the side effects of cutaneous fever and flushing due to excessive release of niacin.

Hereinafter, the present invention will be described in more detail with reference to Examples. However, these Examples are for the illustrative purpose only, and the invention is not intended to be limited by these Examples.

### [Mode for Invention]

### Example 1: Swelling test for types of polymer base

A swelling test for the types of polymer base was performed to observe whether the niacin formulation of the present invention releases the drug for a desired time period, and maintains its matrix shape during drug release.
Specifically, niacin formulations were prepared according to the following Preparation Examples 1 to 4.

### Preparation Example 1 (No. 46)

500.0 mg of niacin as a drug, 90 mg of lactose as an excipient, and 90 mg of microcrystalline cellulose were mixed well to increase the fluidity of drug. 170 mg of HPMC 2208 (100, 000 cps) as a polymer base were added to a powder mixer, and mixed homogeneously. Then, 0.01 ml of ethanol was sprayed to prepare wet granules.
The prepared granules were dried in an oven at 60C, and then evenly milled. Then, 16 mg of magnesium stearate was additionally mixed for molding of the formulation. A niacin-containing tablet was tableted and prepared using a rotary tablet machine.

### Preparation Example 2 (No. 47)

A niacin-containing tablet was tableted and prepared in the same manner as in Preparation Example 1, except that 15 mg of sodium alginate was used as a polymer base, in addition to the composition in Preparation Example 1.

### Preparation Example 3 (No. 48)

A niacin-containing tablet was tableted and prepared in the same manner as in Preparation Example 1, except that 17 mg of Carbopol 971 NF was used as a polymer base, in addition to the composition in Preparation Example 1.

### Preparation Example 4 (No. 49)

A niacin-containing tablet was tableted and prepared in the same manner as in Preparation Example 1, except that 17 mg of Povidone K-30 was used as a polymer base, in addition to the composition in Preparation Example 1.
The compositions of the niacin-containing oral formulations according to Preparation Examples are summarized in the following Table 1.

**[Table 1]**

| Composition of niacin-containing oral formulation (mg) | | | | | | | | |
|---|---|---|---|---|---|---|---|---|
| No. | Niacin | Microcrystalline cellulose | Lactose | HPMC 2208 | Sodium alginate | Carbopol 971NF | Povidone K-30 | Magnesium stearate |
| Preparation Example 1 (No. 46) | 500.0 | 90 | 90 | 170 | - | - | - | 16 |
| Preparation Example 2 (No. 47) | 500.0 | 90 | 90 | 170 | 15 | - | - | 16 |
| Preparation Example 3 (No. 48) | 500.0 | 90 | 90 | 170 | - | 17 | - | 16 |
| Preparation Example 4 (No. 49) | 500.0 | 90 | 90 | 170 | - | - | 17 | 16 |

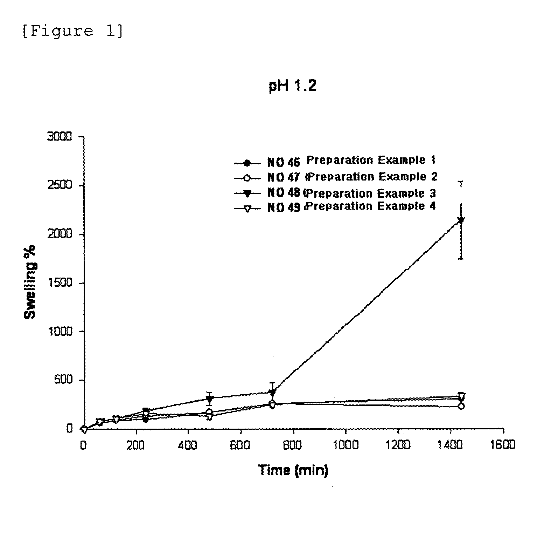

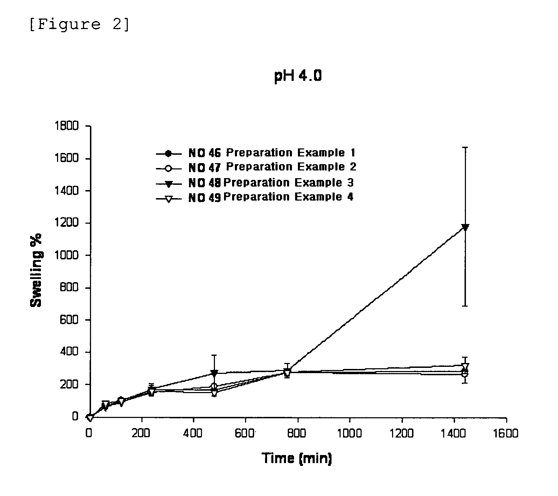

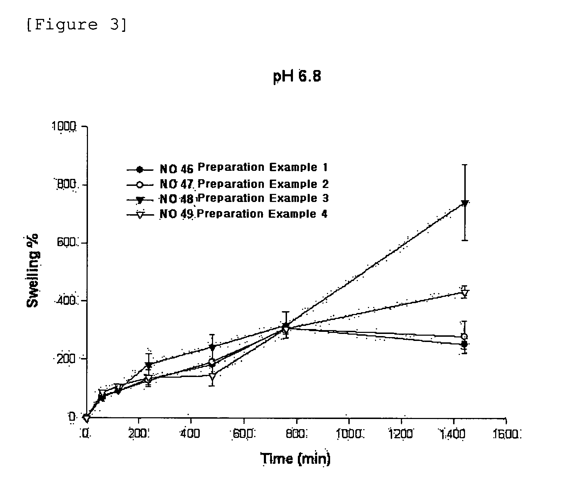

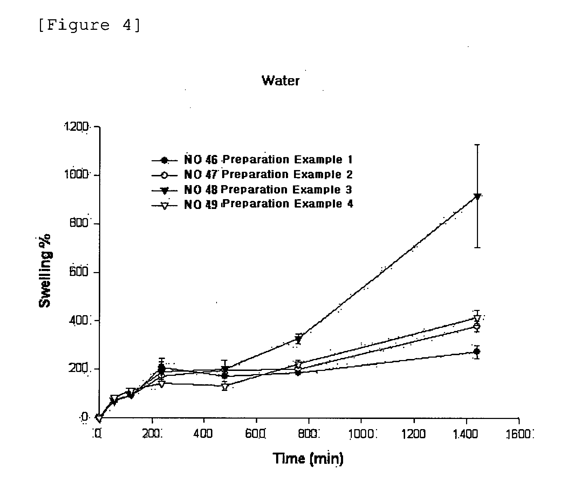

Subsequently, swelling test was performed using the niacin formulations prepared in Preparation Examples 1 to 4. First, water, artificial gastric juice (pH 1.2), an acetic acid buffer solution (pH 4.0), and artificial intestinal juice (pH 6.8) were prepared as a dissolution medium, and then a weight of sinker before and after immersed in the dissolution media was measured to determine the water absorption amount. Then, the weight of each niacin tablet prepared in Preparation Examples 1 to 4 was measured, and swollen in 900 ml of the dissolution media. The procedure was performed at each time point, and then the weight of swollen tablet was measured. Subsequently, the swollen tablets were dried in an oven at 80C° for 24 hrs. The weight of the dried tablet was measured to calculate swelling% and erosion (Figs. 1 to 4).
As a result, the volume of the tablet, prepared using HPMC and Carbopol 971 NF (carboxyvinyl polymer) in Preparation Example 3, was significantly increased in all dissolution media in a time-dependent manner, whereas the tablets prepared using other polymer bases are hardly swollen, except the increased volume due to water absorption. Thus, it can be seen that the tablet prepared in Preparation Example 3 has an excellent swelling ability. Consequently, when a niacin formulation is prepared using a predetermined ratio of HPMC to carboxyvinyl polymer, the niacin formulation has a remarkably increased swelling ability due to its water absorption, as compared to niacin formulations containing other polymer bases.

### Example 2: Comparative dissolution test for commercial formulation and formulation of the present invention

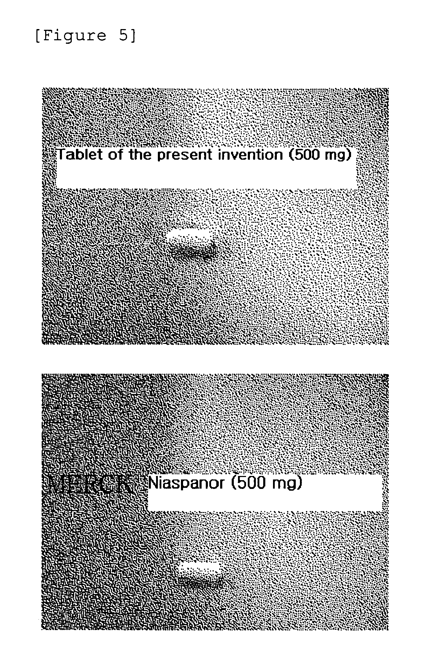

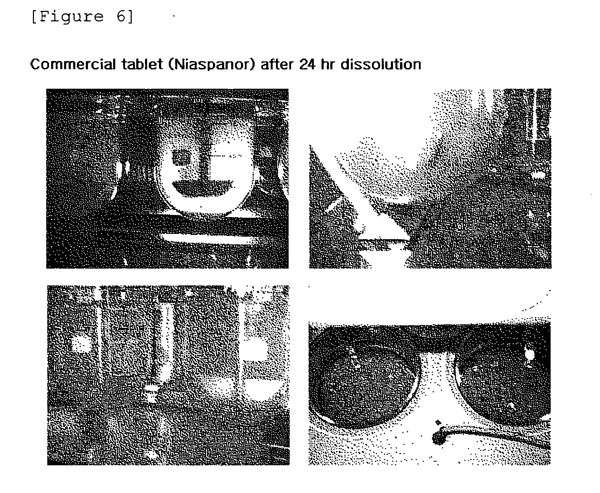

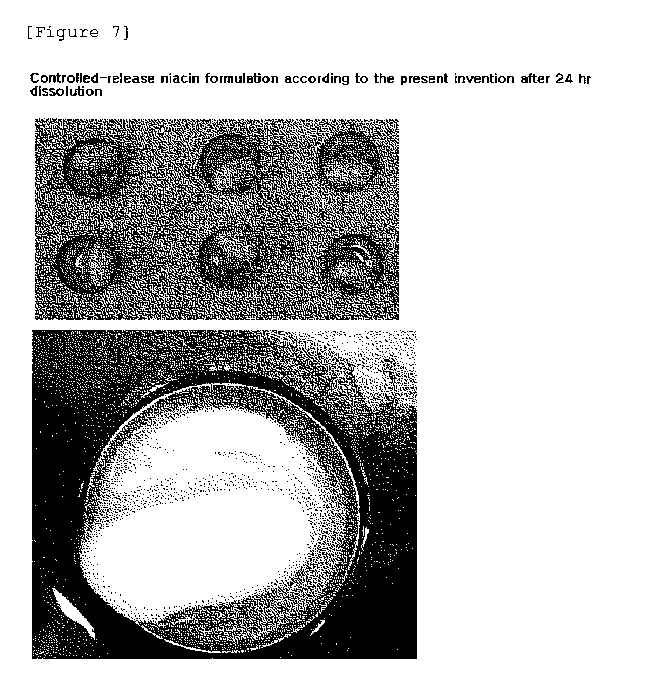

A dissolution test was performed to confirm whether the controlled-release niacin formulation of the present invention and a commercial sustained-release formulation maintain their matrix shape during a desired time period (24 hr or longer) or not. The controlled-release niacin formulation containing 500 mg of niacin according to the present invention (composition of 500 mg of niacin, 90 mg of lactose, 90 mg of microcrystalline cellulose, 170 mg of HPMC, and 16 mg of magnesium stearate) and a commercial sustained-release niacin formulation, Niaspanor™ were subjected to the dissolution test in 900 ml of water at 37C° and 50 rpm for 24 hrs. The dissolution test was performed using a dissolution tester, PT4005956 manufactured by Pharma test (Germany), and a paddle method of United States Pharmacopeia (USP) dissolution test II. Then, the samples were taken, and the matrix shape of each sample was observed (Figs. 5 to 7). As a result, it was found that the controlled-release niacin formulation of the present invention was swollen to maintain its matrix shape for 24 hrs, whereas the commercial sustained-release niacin formulation, Niaspanor™ was gradually eroded, and completely disintegrated and dispersed after 24 hrs.

### Example 3: Change in matrix shape of niacin formulation according to composition of carboxyvinyl polymer and HPMC

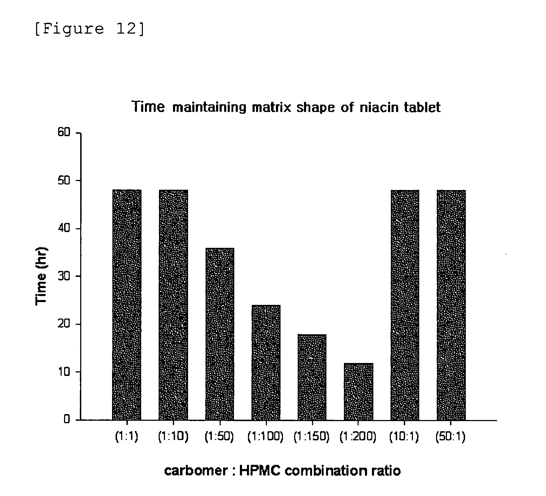

A dissolution test on niacin formulations prepared by varying the composition ratio of carboxyvinyl polymer to HPMC was performed, to observe the matrix shape of each niacin formulation. Specifically, niacin tablets (basic composition: 500 mg of niacin, 90 mg of lactose and 16 mg of magnesium stearate) containing carboxyvinyl polymer and HPMC in a weight ratio of 1:1, 1:10, 1:50, 1:100, 1:150, 1:200, 10:1, and 50:1 were prepared, respectively. The prepared tablets were subjected to the dissolution test in 900 ml of water at 37C° and 50 rpm for 48 hrs. The dissolution test was performed in the same manner as in Example 2. The matrix shape of each formulation was observed with the naked eye at each dissolution time point. In the case where the round shape was observed, the formulation was regarded to maintain its matrix shape. The results are shown in Fig. 12.

As shown in Fig. 12, in the case where the niacin formulation contains the carboxyvinyl polymer and HPMC in a weight ratio of 50:1 to 1:100, its matrix shape was constantly maintained for 24 hr or longer. However, in the case where the weight ratio of carboxyvinyl polymer to HPMC is not within the above range, the time maintaining the matrix shape was dramatically reduced. In addition, in the case where the weight ratio of carboxyvinyl polymer to HPMC is 50:1 or 10:1, it was found that the matrix shape was maintained, but the dissolution pattern of niacin was significantly variable, thereby not exhibiting the dissolution rate suitable for the treatment of hyperlipidemia (data not shown). Accordingly, when the niacin formulation contains the carboxyvinyl polymer and HPMC at a ratio of 1:1 to 1:100, the matrix shape of the niacin tablet is maintained and a suitable dissolution pattern is exhibited. If the ratio is not within the above range, the niacin tablet does not maintain its matrix shape for a desired time period, or even if maintaining its matrix shape, a suitable dissolution rate is hardly obtained.

### Example 4: Change in dissolution rate according to type and content of HPMC

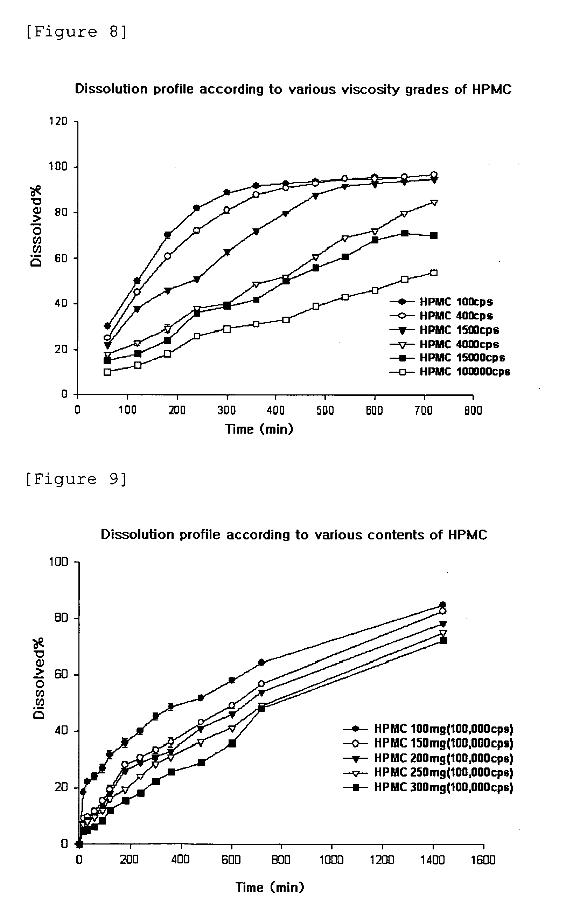

The type of HPMC is classified by the type of substituent, viscosity grade, SR (sustained release), ratio of methoxyl to hydroxypropyl, or the like.
First, the present inventors determined dissolution profiles according to viscosity of HPMC. In particular, each niacin tablet was prepared according to the following composition: 500 mg of niacin, 50 mg of lactose, 5 mg of magnesium stearate (lubricant), and 200 mg of HPMC, with the proviso that each niacin tablet was prepared by varying the labeled viscosity of HPMC (100, 400, 1500, 4000, 15000 and 100000). Then, the prepared tablets were subjected to the dissolution test in 900 ml of water at 37C° and 50 rpm at each time period. The results are shown in Fig. 8. The dissolution test was performed in the same manner as in Example 2.

Second, the present inventors determined dissolution profiles according to content of HPMC. In particular, each niacin tablet was prepared according to the following composition: 500 mg of niacin, 50 mg of lactose, 10 mg of magnesium stearate, and HPMC having a viscosity of 100000 cps, with the proviso that each niacin tablet was prepared by varying the content of HPMC from 100 to 300 mg (100, 150, 200, 250 and 300 mg) . Then, the prepared tablets were subjected to the dissolution test in 900 ml of water at 37C° and 50 rpm at each time period. The results are shown in Fig. 9. The dissolution test was performed in the same manner as in Example 2.

As shown in Figs. 8 and 9, it can be seen that the dissolution rate of the niacin formulation does not depend on the content of HPMC, but changes depending on the viscosity of HPMC. In the case of using HPMC having a viscosity of 100000 cps, the niacin formulation exhibits a suitable dissolution rate, considering its efficacy and side effects.

### Example 5: Change in dissolution rate according to content of carboxyvinyl polymer

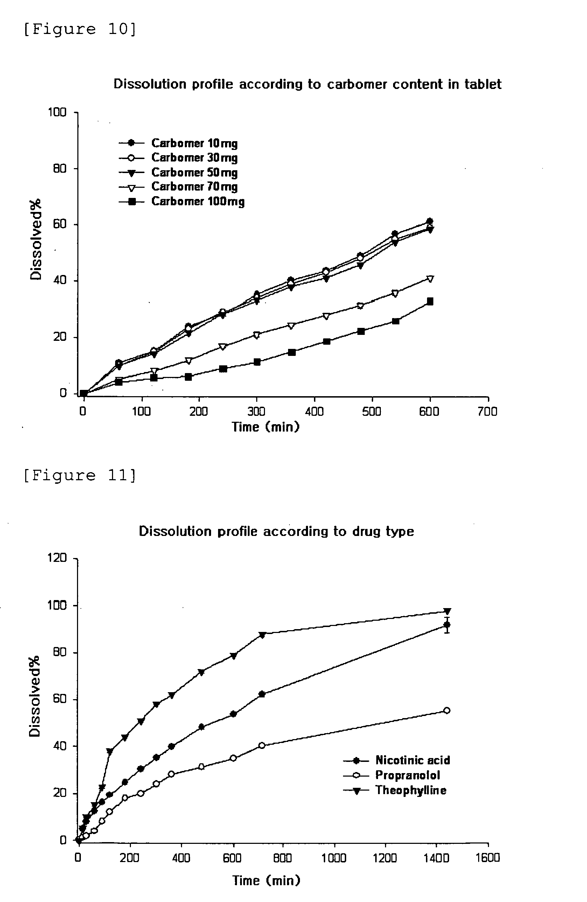

To observe the change in dissolution rate according to content of carboxyvinyl polymer, the present inventors prepared each niacin tablet according to the following composition: 500 mg of niacin, 50 mg of lactose, 10 mg of magnesium stearate, and carbomer (carboxyvinyl polymer), with the proviso that each niacin tablet was prepared by varying the content of carbomer from 10 to 100 mg. Then, the prepared tablets were subjected to the dissolution test in 900 ml of artificial intestinal juice (pH 7.5) at 37C° and 50 rpm at each time period. The results are shown in Fig. 10. The dissolution test was performed in the same manner as in Example 2, except using artificial intestinal juice as a dissolution medium.
As shown in Fig. 10, it can be seen that the dissolution rate of niacin formulation changes depending on the content of carbomer.

### Example 5: Dissolution profile according to type of drug

To confirm whether the composition ratio of carboxyvinyl polymer to HPMC according to the present invention is applied to other drugs, in addition to niacin, a dissolution test was performed according to type of drug. In particular, each formulation was prepared according to the following composition: 90 mg of lactose, 90 mg of microcrystalline cellulose, and 170 mg of HPMC, and 16 mg of magnesium stearate, with the proviso that each formulation was prepared using a different active ingredient (niacin (500 mg), propranolol (50 mg) and theophylline (50 mg), respectively). Then, the prepared formulations were subjected to the dissolution test in 900 ml of water at 37C° and 50 rpm at each time period. The results are shown in Fig. 11. The dissolution test was performed in the same manner as in Example 2.

As shown in Fig. 11, it was found that even though having the same ratio of HPMC to carboxyvinyl polymer, each formulation has different dissolution pattern depending on the type of active ingredient, and only the niacin formulation exhibits the dissolution pattern being required for better efficacy and fewer side effects. Consequently, it can be seen that the composition ratio of HPMC to carboxyvinyl polymer, which is invented to determine dissolution profiles for minimizing the side effect of niacin, is specifically applied to niacin.

### [Industrial Applicability]

As described above, the controlled-release niacin formulation according to the present invention maintains its matrix shape until completion of release, and maintains its release pattern without fluctuation for a desired time period, unlike a commercial formulation. In particular, since niacin formulations are used for long-term treatment of hyperlipidemia, the controlled-release niacin formulation of the present invention, capable of maintaining effective blood concentration and high stability for a long period of time, is very useful.
In addition, the controlled-release niacin formulation of the present invention is an oral formulation which can be readily mass-produced by a conventional process without additional equipment, thereby being an alternative to commercial tablets.

## Claims

1. A controlled-release niacin formulation comprising niacin; hydroxypropyl methylcellulose; and a carboxyvinyl polymer, wherein the carboxyvinyl polymer and hydroxypropyl methylcellulose are contained in a weight ratio of 1:1 to 1:100.

2. A controlled-release niacin formulation comprising niacin; hydroxypropyl methylcellulose; and a carboxyvinyl polymer, wherein the carboxyvinyl polymer and hydroxypropyl methylcellulose are contained in a weight ratio of 1:1.5 to 1:20.

3. The controlled-release niacin formulation according to claim 1 or 2, comprising one or more ingredients selected from the group consisting of an additive, a disintegrant and a lubricant.

4. The controlled-release niacin formulation according to claim 3, further comprising a binder.

5. The controlled-release niacin formulation according to claim 3, wherein the disintegrant is selected from the group consisting of croscamellose sodium, sodium starch glycolate, pregelatinized starch, microcrystalline cellulose, crospovidone, and other commercially available polyvinylpyrrolidone, low substituted hydroxypropylcellulose, alginic acid, carboxymethylcellulose calcium salts and sodium salts, colloidal silicon dioxide, guar gum, magnesium aluminium silicate, methyl cellulose, powdered cellulose, starch, sodium alginate, and mixtures thereof.

6. The controlled-release niacin formulation according to claim 3, wherein the lubricant is selected from the group consisting of magnesium stearate, silica oxide, colloidal silicon dioxide, talc, and mixtures thereof.

7. The controlled-release niacin formulation according to claim 1 or 2, wherein the hydroxypropyl methylcellulose has a viscosity of 80,000 to 120,000 cps.

8. A method for preparing a controlled-release niacin formulation, comprising the steps of
(a) mixing niacin; hydroxypropyl methylcellulose; a carboxyvinyl polymer; an additive; and a disintegrant;
(b) preparing granules by adding a liquid solvent; and
(c) mixing the granules with a lubricant to perform tableting, wherein the carboxyvinyl polymer and hydroxypropyl methylcellulose are mixed in a weight ratio of 1:1 to 1:100.

9. A method for preparing a controlled-release niacin formulation, comprising the steps of
(a) mixing niacin; hydroxypropyl methylcellulose; a carboxyvinyl polymer; an additive; and a disintegrant;
(b) preparing granules by adding a liquid solvent; and
(c) mixing the granules with a lubricant to performtableting, wherein the carboxyvinyl polymer and hydroxypropyl methylcellulose are mixed in a weight ratio of 1:1.5 to 1:20.

10. The method for preparing a controlled-release niacin formulation according to claim 8 or 9, further comprising the step of mixing with a binder in step (a).

11. The method for preparing a controlled-release niacin formulation according to claim 8 or 9, wherein the liquid solvent is selected from the group consisting of water, ethanol, isopropyl alcohol, glycerin, propylene glycol, polyethylene glycol, and mixed solvents thereof.

12. The method for preparing a controlled-release niacin formulation according to claim 11, wherein the liquid solvent is water or a mixed solvent of water and ethanol, and is used in an amount of 10 to 30% by weight, based on the weight of niacin.
